# EUROPEAN PATENT SPECIFICATION

(11) **EP 3 641 650 B1**
(45) Date of publication and mention of the grant of the patent: **18.11.2020**
(21) Application number: 18733255.6
(22) Date of filing: 21.06.2018
(51) Int. Cl.: G16H 30/20

(54) **METHOD AND SYSTEM FOR IMAGE ANALYSIS OF A MEDICAL IMAGE**
VERFAHREN UND SYSTEM ZUR BILDANALYSE EINES MEDIZINISCHEN BILDES
PROCÉDÉ ET SYSTÈME D'ANALYSE D'IMAGES MÉDICALES

(30) Priority: 23.06.2017 EP 17177569
(43) Date of publication of application: 29.04.2020
(73) Proprietor: Koninklijke Philips N.V., 5656 AG Eindhoven (NL)
(72) Inventor: CHAKRABARTI, Biswaroop, 5656 AE Eindhoven (NL); RAGHOTHAM VENKAT, Prasad, 5656 AE Eindhoven (NL); BHAT, Ravindra, 5656 AE Eindhoven (NL)
(74) Representative: Philips Intellectual Property & Standards
(86) International application number: PCT/EP2018/066621
(87) International publication number: WO 2018/234476

(56) References cited:
- US-A1- 2009 067 700
- US-A1- 2010 086 182
- US-A1- 2010 135 562
- US-A1- 2014 324 469
- US-A1- 2016 124 619

## Description

### FIELD OF THE INVENTION

The present invention relates to a method and system for image analysis of a medical image, a computer program; as defined in the independent claims.

### BACKGROUND OF THE INVENTION

In modern medicine, medical imaging has undergone major advancements. It is a non-invasive technique used to gather visual representations of the interior of a body for clinical analysis and medical intervention. Medical imaging is furthermore used to gather visual representations concerning the functions of organs and tissues. It seeks to reveal internal structures hidden by the skin and bones, as well as to diagnose and treat diseases.

Medical imaging refers both to conventional images as well as to moving images, i.e. videos, to two-dimensional images, but also to three-dimensional ones. Medical imaging techniques comprise, for example, x-ray imaging, especially computed tomography imaging, ultrasound imaging and magnetic resonance imaging.

In the clinical context, medical imaging is generally equated to radiology and the medical practitioner responsible for acquiring and interpreting the images is a radiologist.

Reliable imaging is of paramount importance for a radiologist's decision making and can reduce unnecessary procedures. Surgical interventions, for example, may be avoided altogether if diagnostic imaging services such as x-ray imaging are available.

However, proper decision making presupposes high quality medical images. While said images themselves may be of high quality, there might still be the problem that a radiologist or other medical professional does not have access to an adequate display. Such a situation may, for instance, occur in a tele-radiology or brokered service setup, where multiple readers try to interpret a certain medical image independently from each other using their respective displays on mobiles or tablets and the like. The same situation applies to a busy radiologist on the move who gets images referred to his/her smartphone in order to provide a second opinion on a medical diagnosis.

In these cases, due diligence of a medical diagnosis can no longer be assured. As a result, medical negligence may be induced and at worst wrong treatment. Furthermore, urgent medical treatments may have to be postponed.

Document US2010086182 A1 may be considered to disclose a method for image analysis of a medical image, said method comprising: receiving a medical image and first region of interest, ROI, information indicating a first ROI within said medical image receiving second ROI information indicating a second ROI within said medical image and viewing data including display data with which the second ROI has been displayed on a display and checking the quality of by checking the viewing data.

### SUMMARY OF THE INVENTION

It is an object of the present invention to provide a method and system for image analysis of a medical image, which assures due diligence of a corresponding medical diagnosis.

It is a further object of the present invention to provide a method and device for providing annotations of a medical image.

In a first aspect of the present invention a method for image analysis of a medical image is presented which comprises
- receiving a medical image, first annotations of said medical image and first region of interest, ROI, information indicating a first ROI within said medical image.
- receiving second annotations of said medical image, second ROI information indicating a second ROI within said medical image and viewing data including display data and/or viewing time with which the second ROI has been displayed on a display and based on which the second annotations have been made, and
- checking the quality of the second annotations by comparing the second ROI information to the first ROI information and by checking the viewing data.

In a further aspect of the present invention a system for image analysis of a medical image is presented which comprises
- a first input unit configured to receive a medical image, first annotations of said medical image and first region of interest, ROI, information indicating a first ROI within said medical image,
- a second input unit configured to receive second annotations of said medical image, second ROI information indicating a second ROI within said medical image and viewing data including display data and/or viewing time with which the second ROI has been displayed on a display and based on which the second annotations have been made, and
- a quality check unit configured to check the quality of the second annotations by comparing the second ROI information to the first ROI information and to check the viewing data.

In a further aspect of the present invention, there is provided a computer program which comprises program code means to carry out the steps of the method for image analysis when said computer program is carried out on the computer as well as a non-transitory computer-readable recording medium that stores therein a computer program product, which, when executed by a processor, causes the method disclosed herein to be performed.

Preferred embodiments of the invention are defined in the dependent claims.

The present invention is based on the idea to assure due diligence of a medical diagnosis based on analysis of a medical image.

During analysis of medical images readers do not always have access to adequate displays. In this situation medical professionals are usually forced to base their medical diagnosis solely on badly displayed images, e.g. images which are only displayed with low resolution and/or on small screens.

This problem particularly arises in tele-radiology or brokered service setups and when a second opinion concerning a diagnosis is required, i.e. in cases where clinical diagnosis or treatment are already difficult. As a consequence, the risk of false diagnoses or even false treatment and treatment delays is greatly increased.

The method for image analysis according to the present invention may solve these problems. In particular, said method assures that a radiologist's diagnosis based on the analysis of a medical image on a display has been assessed carefully and correctly. In cases, for example, where lesions have already been detected by a previous reader, it is essential that the correct region of an image, the ROI, is brought in the viewport of the current reader's displaying device. Only then the previous reader's annotations can be understood correctly. Moreover, for satisfactory inspection a medical image may need to be displayed according to viewing data transmitted from the previous reader. Otherwise the likelihood of detecting a lesion or other medical anomaly is at best suboptimal.

By introducing a method for image analysis of a medical image wherein the quality of the second reader's diagnosis is checked, false diagnoses can be reduced to a minimum and false treatments or treatment delays can be avoided.

In an embodiment of the method for image analysis, checking the viewing data comprises checking if the viewing data meet a viewing data requirement. In order to check the quality of a reader's diagnosis it has to be checked not only whether he/she has selected the ROI as found by a previous or parallel reader but also whether his/her viewing data meet a viewing data requirement. Said requirement is usually specified by the previous or parallel reader. However, viewing data requirements may also be determined automatically by a computer program analyzing the image.

In another embodiment of the method for image analysis, the viewing data may further include zoom level and/or panning level. In a further embodiment the display data may further include resolution and/or display size and/or the number of pixels in the screen and/or in the second ROI. That is, it is precisely monitored how a radiologist displays a medical image on his/her displaying device. Furthermore, the kind of display used is analyzed. It may, for example, be recorded that for the first 5 seconds of displaying only the upper half of the image is viewed and that the display used has a diagonal of 13 inches and a 1280 x 800 resolution and the ROI covered 1000 pixels.

In an embodiment of the method for image analysis, the viewing data requirement comprises a viewing time threshold and/or a zoom level threshold and/or a panning level threshold and/or a resolution threshold and/or a number of pixel threshold and/or a display size threshold. A radiologist viewing an image only superficially is not very likely to confirm a reliable diagnosis. Therefore, it must be assured that the radiologist views the medical image for a sufficient amount of time. The time threshold for complicated lesions may be higher than the time threshold for simple lesions. The location of small anomalies such as small pieces of cancer tissue may only be detectable and assessable at certain zoom levels. To avoid overlooking of such regions it is important that a certain zoom threshold is complied with. For two-dimensional as well as three-dimensional images a minimum panning may be required. Otherwise a ROI may not be displayed properly. For example, malfunctions of the heart may not be diagnosed from a perspective where the heart is covered by ribs. Likewise, retinal diseases may only be found from a certain perspective. Furthermore, there may exist displays not capable at all to view an image such that a lesion or illness could be diagnosed properly. All zooming, for example, will not help if the resulting resolution is too low to view the ROI with sufficient quality. The same applies if the number of pixels of the display is too low or the display size is too small.

In a further embodiment, the ROI information of the method for image analysis comprises position information and/or size information indicating the position and/or size of the ROI. There exist various forms of ROI information. ROI information may, for example, be directly given by a marking in the medical image, such as a red circle around a supposed bone fracture. ROI information may also be given in the form of coordinates. The ROI information comprises vital information about a patient's injury or illness. Above all the location and size of the region where a patient's suffering originate from provides indispensable information for doctors.

In another embodiment of the method for image analysis, the first annotations are provided by one or more users and/or generated by automated image analysis. They may be provided in advance or in parallel with the second annotations of another reader.

In yet another embodiment, the method for image analysis of a medical image further comprises issuing the result of the quality check, and/or adapting the image according to the quality check. Whether the first and second ROI match or not and whether the viewing data fulfill certain requirements is decisive for the subsequent treatment of a patient. In order to guarantee that the check is passed completely, the outcome of the quality check may be issued to the reader. For example, the result may be displayed on the display or issued via an audio message. In case of deficiencies the reader may adapt the image or even switch to another display. However, a required adaption may also be supported by an automatism or even be performed totally automatically.

In yet a further embodiment, the method for image analysis of a medical image further comprises receiving further annotations of said medical image, further ROI information indicating one or more further ROI within said medical image and further viewing data including display data and/or viewing time with which the further ROI has been displayed on a display and based on which the further annotations have been made, and checking the quality of the further annotations by comparing the further ROI information to the first and /or second ROI information and by checking the further viewing data. In assessing a medical image, it might be necessary to involve a larger group of medical professionals, possibly located all over the world. Each doctor will then determine a respective ROI and make respective annotations. In order to then judge which, diagnose is correct, all ROIs need to be compared and respective viewing data need to be checked accordingly. For instance, the positions of all image portions displayed will be recorded and compared against each other. Furthermore, the time corresponding to each viewport view may be recorded and matched against a predetermined time-threshold.

In an embodiment of the image analysis method, the first annotations and the first ROI information are based on having viewed the medical image on a high resolution display and the second ROI has been displayed on a low resolution display. That is, the second annotations are based on medical images displayed at low resolution. Since treatment decisions may be solely based on these second annotations the quality check of the second annotations is particularly important.

In an embodiment of the system for image analysis of a medical image, the system further comprises an output unit configured to issue the result of the quality check, and/or an adaption unit configured to adapt the image according to the quality check. The output unit may be comprised in the display such that the result of the quality check could directly be displayed on the image. Likewise, the output unit may be configured to issue the result via an audio signal or message. The adaption unit may be configured to adapt the portion of the image viewed. Apart from scrolling or rotating the adaption unit may also be configured to change the zoom level. In order to display three-dimensional images, the adaption unit may also possess a panning function.

In another embodiment of the system, the second input unit is further configured to receive further annotations of said medical image, further ROI information indicating one or more further ROI within said medical image and further viewing data including display data and/or viewing time with which the further ROI has been displayed on a display and based on which the further annotations have been made, and to check the quality of the further annotations by comparing the further ROI information to the first and/or second ROI information and to check the further viewing data. Said system could be a tele-radiology system or a brokered service system where three or more radiologists or other doctors assess a medical image with their respective displaying devices.

In a further aspect a method for recording image-related information related to a medical image is presented which comprises
- receiving the medical image,
- displaying the medical image on a display,
- recording annotations made for said medical image,
- detecting and recording region of interest, ROI, information indicating a ROI within said medical image with which the medical image is displayed and based on which the annotations are made, and
- detecting and recording viewing data including viewing time with which the ROI is displayed and based on which the annotations are made.

In yet a further aspect a device for recording image-related information related to a medical image is presented, said device comprising:
- a receiver configured to receive the medical image,
- a display configured to display the medical image, and
- a detection and recording unit configured to
   - record annotations made for said medical image,
   - detect and record region of interest, ROI, information indicating a ROI within said medical image with which the medical image is displayed and based on which the annotations are made, and
   - detect and record viewing data including viewing time with which the ROI is displayed and based on which the annotations are made.

By means of the method for recording image-related information and the device the viewing patterns of a user watching a medical image can be monitored. Apart from ROI information, display data and viewing time data zoom level and/or panning level data may also be recorded. The annotations made may then be evaluated in the light of the viewing pattern monitored. The device may particularly be used as second input unit of the system for image analysis of a medical image.

### BRIEF DESCRIPTION OF THE DRAWINGS

These and other aspects of the invention will be apparent from and elucidated with reference to the embodiment(s) described hereinafter. In the following drawings
Fig. 1 shows a flow chart of a first embodiment of a method for image analysis of a medical image according to the present invention,
Fig. 2 shows a flow chart of a second embodiment of a method for image analysis of a medical image according to the present invention,
Fig. 3 shows a schematic diagram of a first embodiment of a system for image analysis of a medical image according to the present invention,
Fig. 4 shows a schematic diagram of a second embodiment of a system for image analysis of a medical image according to the present invention,
Fig. 5A, 5B and 5C show a medical image as viewed on different displays at different zoom levels,
Figs. 6A, 6B and 6C show a medical image with a zooming guidance for proper displaying, and
Fig. 7 shows a schematic diagram of an embodiment of a device for recording image-related information related to a medical image.

### DETAILED DESCRIPTION OF THE INVENTION

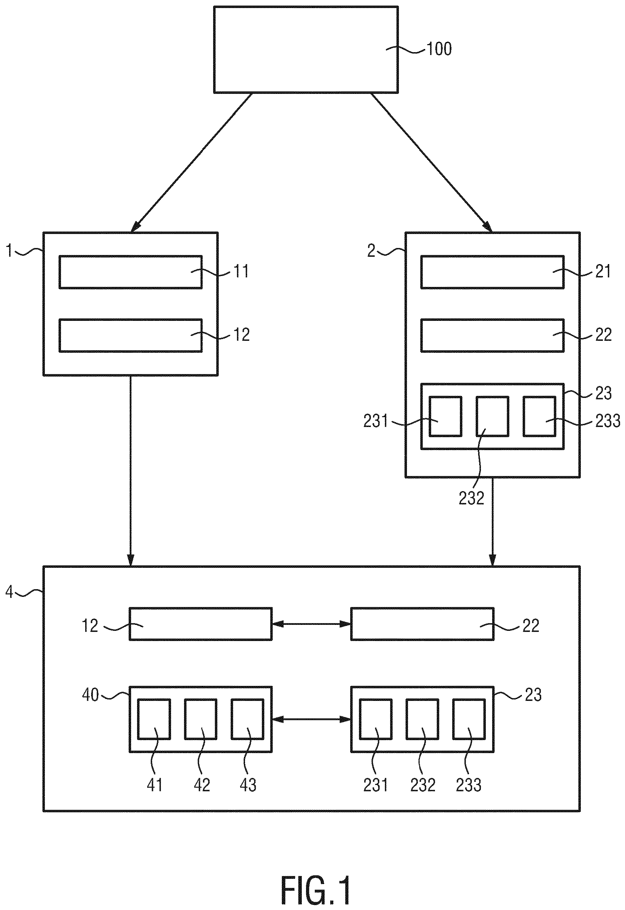

Fig. 1 shows a flow chart of a first embodiment of a method for image analysis of a medical image according to the present invention. Provided a medical image 100 a first reader 1 makes first annotations 11 of the medical image 100 and records first ROI information 12. The first annotations 11 may represent a preliminary diagnosis, patient information or time and date information, for example. A second reader 2, to whom said medical picture 100 is forwarded as well, makes second annotations 21 of the medical image 1 and records second ROI information 22. Furthermore, viewing data 23 are created by the second reader 2. In this embodiment the viewing data 23 comprise zoom level data 231, panning level data 232 and display resolution data 233. The data from the first reader 1 and second reader 2 are then used for a quality check 4 of the second annotations 21, wherein the second ROI information 22 is compared against the first ROI information 12. The quality check 4 will not be passed if the information of the two ROI does not match. Furthermore, it is controlled whether the viewing data 23 fulfill a predetermined viewing data requirement 40, i.e. predetermined viewing data thresholds. In this embodiment the viewing data requirement 40 comprises a zoom level requirement 41, a panning level requirement 42 and a display resolution requirement 43.

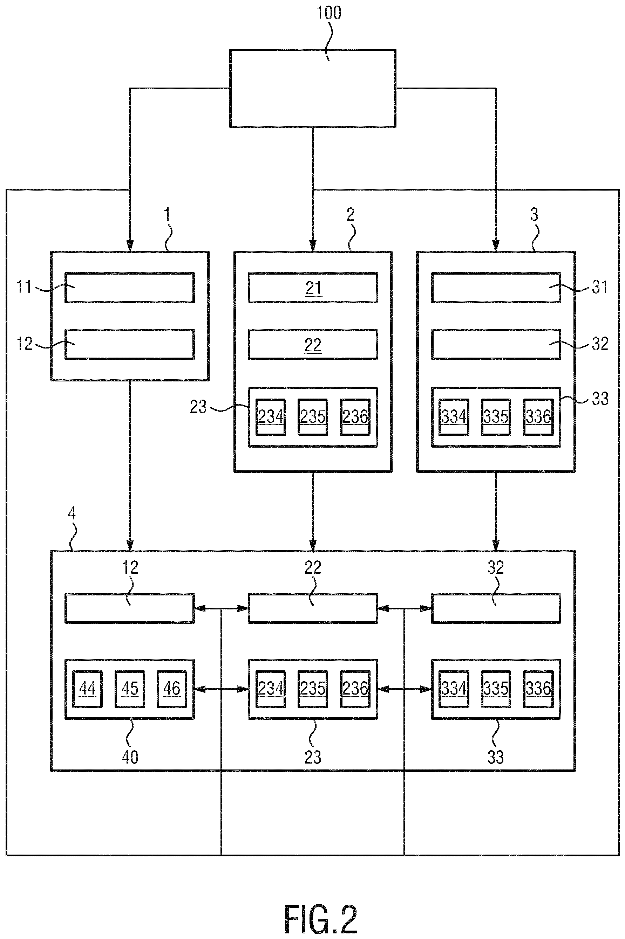

Fig. 2 shows a flow chart of a second embodiment of a method for image analysis of a medical image according to the present invention. Provided a two-dimensional medical image 100 a first reader 1 makes first annotations 11 of the medical image 100 and records first ROI information 12. Given a tele-radiology setup a second reader 2 makes second annotations 21 of the medical image 100 at the same. Apart from this, the second reader 2 also records second ROI information 22 and second viewing data 23, wherein the second viewing data 23 include second viewing time data 234, second number of pixel data 235 and second display size data 236 corresponding to his/her display. In this embodiment, a third reader 3, being part of the setup as well, records third annotations 31, third ROI information 32, and third viewing data 33 comprising third viewing time data 334, third number of pixel data 335 and third display size data 336 corresponding to third reader's display. The data from the first reader 1, second reader 2 and third reader 3 are subsequently used for a quality check 4 of the third annotations 31 and second annotations 21, wherein the third ROI information 32 is compared against the second ROI information 22 and first ROI information 12. It is checked further whether the viewing data 23 and 33 fulfill a predetermined viewing data requirement 40 and/or whether the data 23 and 33 match. In particular, said viewing data requirement 40 comprises a viewing time threshold 44. It may, for example, be required that a radiologist views the medical image for at least five minutes. In this embodiment the viewing data requirement 40 further comprises a number-of-pixels threshold 45 and a display size threshold 46. In fact, the number-of-pixels threshold 45 and the display size threshold 46 may be combined to a display resolution threshold. The result of the quality check 4 is then issued to all readers 1, 2 and 3 so they can adapt their viewports in case the quality check 4 has not been passed.

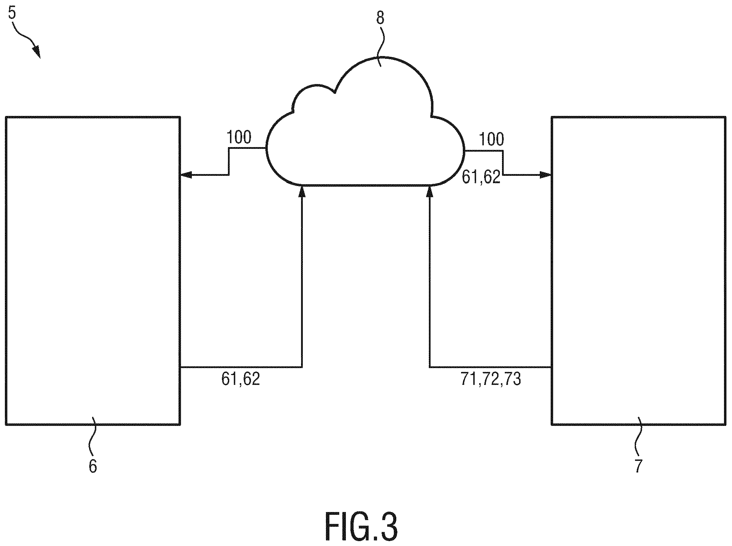

Fig. 3 shows a schematic diagram of a first embodiment of a system 5 for image analysis of a medical image according to the present invention. The system 5 comprises a first input unit 6, a second input unit 7 and a quality check unit 8. In this embodiment, the quality check unit 8 provides a medical image 100 to the first input unit 6 and to the second input unit 7. The first input unit 6 then makes first annotations 61 on the medical image using automated image processing. These annotations and first ROI information 62, which are also gathered automatically in this embodiment, are then forwarded to the quality check unit 8. The quality check unit 8 stores these data and passes them on to the second input unit 7. A radiologist may then use the second input unit 7 to make up his own mind and to assess the image analysis of the first input unit. In order to so, he/she receives the image 100, the first annotations 61 and the first ROI information 62 from the quality check unit 8. After studying the image, the first annotations 61 and first ROI information 62 the radiologist denotes which lesion he/she presumes and also the size and position of the lesion suspected. That is, the radiologist enters second annotations 71 and second ROI information 72 in the second input unit 7. The second input unit 7 also records the radiologist's viewing data 73. Together with the second annotations 71 and the second ROI information 72 these data are then sent back to the quality check unit 8, which performs a quality check on the second annotations 71. If the second annotations 71 are deemed to be based on a defective review, a third input unit may be required.

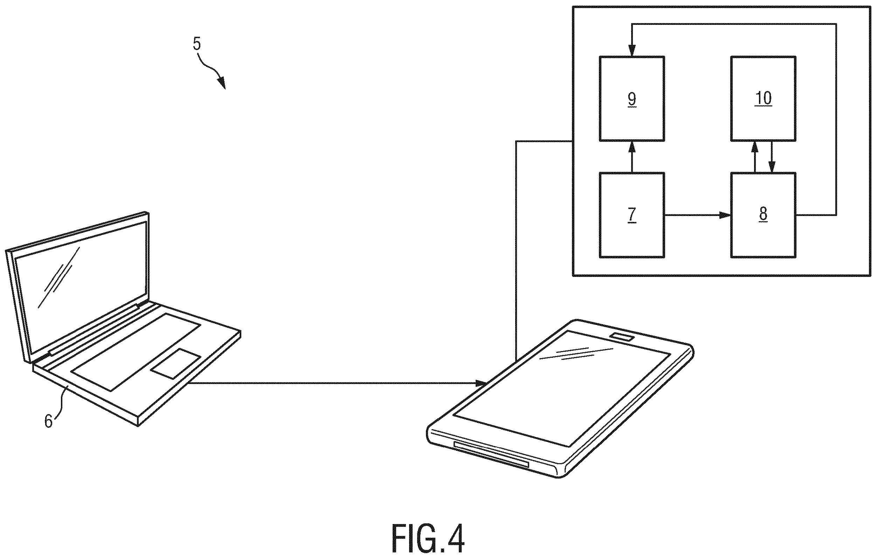

Fig. 4 shows a schematic diagram of a second embodiment of a system 5 for image analysis of a medical image according to the present invention. The system 5 comprises a first input unit 6, a second input unit 7, a quality check unit 8, an output unit 9 and an adaption unit 10. In this embodiment, the second input unit 7, the quality check unit 8, the output unit 9 and the adaption unit 10 are comprised within a single device, wherein said device is a smartphone. The second input unit 7 receives the medical image plus related data from the first input unit 6 and forwards the image to the output unit 9, namely the smartphone's display. At the same time, the second input unit 7 collects second annotations, second ROI information and viewing data and forwards these to the quality check unit 8, which subsequently checks the quality of the second annotations using the data from the first input unit 6. The result of this quality check 4 is then directly presented to a user by means of the display 9. According to the information displayed, i.e. according to the quality check 4, the user may then adapt the medical image by using the adaption unit 10. After the adaption, the quality check unit 8 controls whether the image is now displayed according to predefined requirements. These predefined requirements may originate from the quality check unit 8 itself, but may also be provided by the first input unit 6. The result of the checking after the adaption may also be presented to the user on the display 9.

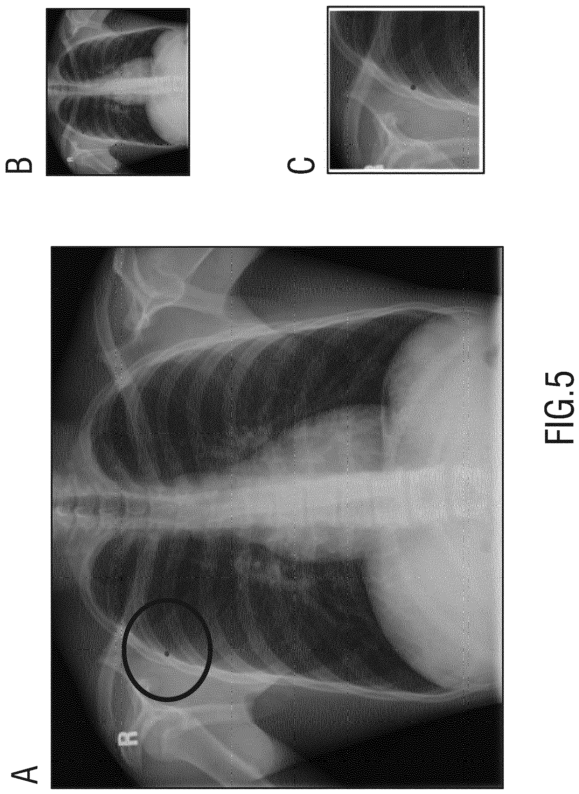

Fig. 5A, 5B and 5C show a medical image as viewed on different displays at different zoom levels. In particular, the figs show a human thorax comprising a clinical anomaly. While Fig. 5A shows the image as viewed on a first device having screen diagonal of approximately 25 inches, the mobile used to display Figs. 5B and 5C has a display diagonal of about 8 inches. That is, the anomaly as viewed in Fig. 5A may be recognized easily, whereas the smaller viewport as shown in Fig. 5B is too small to recognize any anomaly. Hence, a radiologist receiving the image as depicted in Fig. 5B is not capable of making a qualified opinion. However, said small display size suffices if the zoom level is adapted accordingly as shown in Fig. 5C. That is, if the radiologist views the thorax image on an 8 inches display he/she may only assess the anomaly if the zoom level is appropriate. In order to ensure that the radiologist uses a sufficient zoom level the zoom levels used may be recorded by the mobile and sent to a quality check unit.

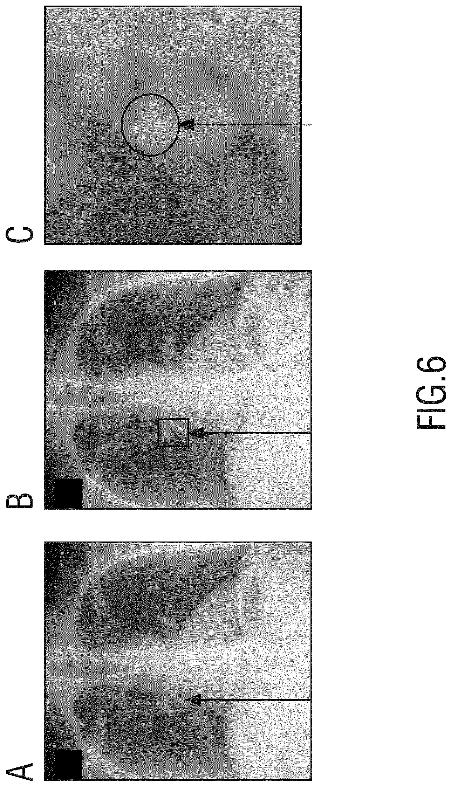

Figs. 6A, 6B and 6C show a medical image with a zooming guidance for proper displaying. In particular, there is shown a human thorax with a lymphadenopathy. Fig. 6A shows the medical image in a form that is not appropriate for diagnosis. However, if ROI information were provided along with the image in accordance with the present invention, a proper diagnosis could be made. Fig. 6B depicts how said ROI information may be transferred, namely by marking around the anomaly in the image. This marking represents a guidance for zooming. Using the ROI marking to find an appropriate zoom level, the lymphadenopathy may be displayed in an optimal manner. The corresponding image is depicted in Fig. 6C.

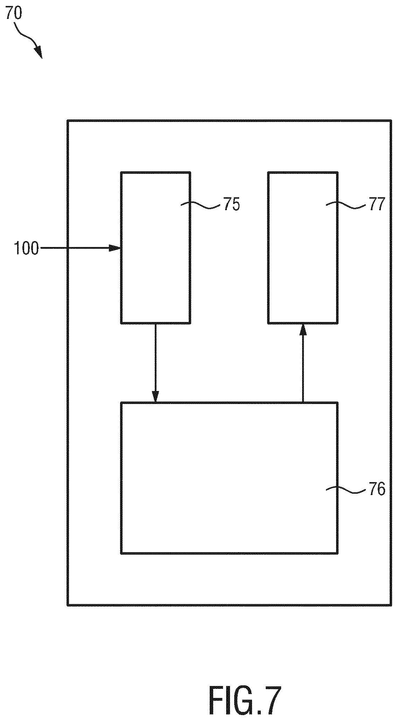

Fig. 7 shows a schematic diagram of an embodiment of a device 70 for recording image-related information related to a medical image 100. The device 70 comprises a receiver 75, a display 76 and a detection and recording unit 77. After the image 100 is received by the receiver 75, the display 76 displays the image 100. On the basis of the image 100 a radiologist subsequently makes annotations. These are recorded by the detection and recording unit 77. The detection and recording unit 77 furthermore detects possible regions of interest and records related information such as coordinates and sizes. Apart from this the viewing data of the radiologist are monitored. For example, it is recorded how long and at what zooming level which area of the image has been viewed on the display 76. In particular, said device may represent the second input unit 7 of the system 5 according to the present invention.

While the invention has been illustrated and described in detail in the drawings and foregoing description, such illustration and description are to be considered illustrative or exemplary and not restrictive; the invention is not limited to the disclosed embodiments.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A method for image analysis of a medical image (100), said method comprising:
- receiving a medical image (100), first annotations (11) of said medical image and first region of interest, ROI, information (12) indicating a first ROI within said medical image (100),
- receiving second annotations (21) of said medical image (100), second ROI information (22) indicating a second ROI within said medical image (100) and viewing data (23) including display data and/or viewing time (234) with which the second ROI has been displayed on a display and based on which the second annotations (21) have been made, and
- checking the quality of the second annotations (21) by comparing the second ROI information (22) to the first ROI information (12) and by checking the viewing data (23).

2. The method according to claim 1,
wherein checking the viewing data (23) comprises checking if the viewing data (23) meet a viewing data requirement (40).

3. The method according to any one of the preceding claims,
wherein the viewing data (23) further include zoom level (231) and/or panning level (232) and/or
wherein the display data include resolution (233) and/or display size (236) and/or number of pixels (235), in particular in the display and/or the second ROI.

4. The method according to claims 2 and 3,
wherein the viewing data requirement 40 comprises a viewing time threshold (44) and/or a zoom level threshold (41) and/or a panning level threshold (42) and/or a resolution threshold (43) and/or a number of pixel threshold (45) and/or a display size threshold (46).

5. The method according to any one of the preceding claims,
wherein said ROI information (12, 22) comprises position information and/or size information indication the position and/or size of the ROI.

6. The method according to any one of the preceding claims,
wherein the first annotations (11) are provided by one or more users and/or generated by automated image analysis.

7. The method according to any one of the preceding claims, further comprising
- issuing the result of the quality check (4), and/or
- adapting the image (100) according to the quality check (4).

8. The method according to any one of the preceding claims, further comprising
- receiving further annotations (31) of said medical image, further ROI information (32) indicating one or more further ROI within said medical image (100) and further viewing data (33) including display data and/or viewing time with which the further ROI has been displayed on a display and based on which the further annotations (31) have been made, and
- checking the quality of the further annotations (31) by comparing the further ROI information (32) to the first and /or second ROI information (12, 22) and by checking the further viewing data (33).

9. The method according to any one of the preceding claims,
wherein the first annotations (11) and the first ROI information (12) are based on having viewed the medical image (100) on a high resolution display and wherein the second ROI has been displayed on a low resolution display.

10. A system (5) for image analysis of a medical image (100), said system comprising:
- a first input unit (6) configured to receive a medical image (100), first annotations (11) of said medical image (100) and first region of interest, ROI, information (12) indicating a first ROI within said medical image (100),
- a second input unit (7) configured to receive second annotations (22) of said medical image (100), second ROI information (22) indicating a second ROI within said medical image (100) and viewing data (23) including display data and/or viewing time (234) with which the second ROI has been displayed on a display and based on which the second annotations (22) have been made, and
- a quality check unit (8) configured to check the quality of the second annotations (21) by comparing the second ROI information (22) to the first ROI information (12) and to check the viewing data (23).

11. The system according to claim 10,
further comprising
- an output unit (9) configured to issue the result of the quality check (4), and/or
- an adaption unit (10) configured to adapt the image (100) according to the quality check (4).

12. The system according to any one of claims 10 to 11,
wherein the second input unit (7) is further configured to receive further annotations (31) of said medical image (100), further ROI information (32) indicating one or more further ROI within said medical image (100) and further viewing data (23) including display data and/or viewing time (334) with which the further ROI has been displayed on a display and based on which the further annotations (31) have been made, and to check the quality of the further annotations (31) by comparing the further ROI information (32) to the first and/or second ROI information (12, 22) and to check the further viewing data (33).

13. A computer program comprising program code means for causing a computer to carry out the steps of the method as claimed in claims 1 to 9.

## Patentansprüche

1. Verfahren zur Bildanalyse eines medizinischen Bildes (100), wobei das Verfahren umfasst:
- Empfangen eines medizinischen Bildes (100), der ersten Anmerkungen (11) des medizinischen Bildes und der Information (12) der ersten Region von Interesse, ROI, die einen ersten ROI innerhalb des medizinischen Bildes (100) anzeigt,
- Empfangen von zweiten Anmerkungen (21) des medizinischen Bildes (100), der zweiten ROI-Informationen (22), die eine zweite ROI innerhalb des medizinischen Bildes (100) angeben, und von Betrachtungsdaten (23), einschließlich Betrachtungsdaten und/oder Betrachtungszeit (234), mit denen die zweite ROI auf einer Anzeige angezeigt wurde, auf deren Grundlage die zweiten Anmerkungen (21) vorgenommen wurden, und
- Überprüfen der Qualität der zweiten Anmerkungen (21) durch Vergleichen der zweiten ROI-Informationen (22) mit den ersten ROI-Informationen (12) und Überprüfen der Betrachtungsdaten (23).

2. Verfahren nach Anspruch 1,
wobei das Überprüfen der Betrachtungsdaten (23) das Überprüfen umfasst, ob die Betrachtungsdaten (23) eine Betrachtungsdatenanforderung (40) erfüllen.

3. Verfahren nach einem der vorhergehenden Ansprüche,
wobei die Betrachtungsdaten (23) ferner den Zoompegel (231) und/oder den Panningpegel (232) umfassen, und/oder
wobei die Betrachtungsdaten eine Auflösung (233) und/oder Anzeigegröße (236) und/oder eine Anzahl von Pixeln (235) umfassen, insbesondere in der Anzeige und/oder der zweiten ROI.

4. Verfahren nach Anspruch 2 und 3,
wobei die Betrachtungsdatenanforderung 40 eine Betrachtungszeitschwelle (44) und/oder eine Zoompegelschwelle (41) und/oder eine Panningpegelschwelle (42) und/oder eine Auflösungsschwelle (43) und/oder eine Anzahl von Pixelschwelle (45) und/oder eine Anzeigegrößenschwelle (46) umfasst.

5. Verfahren nach einem der vorhergehenden Ansprüche,
wobei die ROI-Informationen (12, 22) Positionsinformationen und/oder Größeninformationen umfassen, die die Position und/oder Größe der ROI angeben.

6. Verfahren nach einem der vorhergehenden Ansprüche,
wobei die ersten Anmerkungen (11) von einem oder mehreren Benutzern bereitgestellt und/oder durch automatisierte Bildanalyse erzeugt werden.

7. Verfahren nach einem der vorhergehenden Ansprüche, ferner umfassend
- Ausgabe des Ergebnisses der Qualitätsprüfung (4) und/oder
- Anpassung des Bildes (100) gemäß der Qualitätsprüfung (4).

8. Verfahren nach einem der vorhergehenden Ansprüche, ferner umfassend
- Empfangen weiterer Anmerkungen (31) des medizinischen Bildes, weiterer ROI-Informationen (32), die eine oder mehrere weitere ROI innerhalb des medizinischen Bildes (100) angeben, und weiterer Betrachtungsdaten (33), einschließlich Betrachtungsdaten und/oder Betrachtungszeit, mit denen die weitere ROI auf einem Display angezeigt wurde, und auf deren Grundlage die weiteren Anmerkungen (31) vorgenommen wurden, und
- Überprüfen der Qualität der weiteren Anmerkungen (31) durch Vergleichen der weiteren ROI-Informationen (32) mit den ersten und/oder zweiten ROI-Informationen (12, 22) und Überprüfen der weiteren Betrachtungsdaten (33) .

9. Verfahren nach einem der vorhergehenden Ansprüche,
wobei die ersten Anmerkungen (11) und die ersten ROI-Informationen (12) darauf beruhen, dass das medizinische Bild (100) auf einer hochauflösenden Anzeige betrachtet wurde, und wobei die zweite ROI auf einer niedrigauflösenden Anzeige angezeigt wurde.

10. System (5) zur Bildanalyse eines medizinischen Bildes (100), wobei das System umfasst:
- eine erste Eingabeeinheit (6), die konfiguriert ist, um ein medizinisches Bild (100), erste Anmerkungen (11) des medizinischen Bildes (100) und eine Information (12) der erstes Region von Interesse, ROI, zu empfangen, die eine erste ROI innerhalb des medizinischen Bildes anzeigt (100),
- eine zweite Eingabeeinheit (7), die konfiguriert ist, um zweite Anmerkungen (22) des medizinischen Bildes (100), eine zweite ROI-Information (22), die eine zweite ROI innerhalb des medizinischen Bildes (100) anzeigt, und Betrachtungsdaten (23) einschließlich Betrachtungsdaten und/oder Betrachtungszeit (234) zu empfangen, mit denen die zweite ROI auf einer Anzeige angezeigt wurde, und auf deren Grundlage die zweiten Anmerkungen (22) vorgenommen wurden, und
- eine Qualitätsprüfeinheit (8), die konfiguriert ist, um die Qualität der zweiten Anmerkungen (21) durch Vergleichen der zweiten ROI-Informationen (22) mit den ersten ROI-Informationen (12) zu überprüfen, und um die Betrachtungsdaten (23) zu überprüfen.

11. System nach Anspruch 10, ferner umfassend
- eine Ausgabeeinheit (9), die konfiguriert ist, um das Ergebnis der Qualitätsprüfung (4) auszugeben, und/oder
- eine Anpassungseinheit (10), die konfiguriert ist, um das Bild (100) gemäß der Qualitätsprüfung (4) anzupassen.

12. System nach einem der Ansprüche 10 bis 11,
wobei die zweite Eingabeeinheit (7) ferner konfiguriert ist, um weitere Anmerkungen (31) des medizinischen Bildes (100), weitere ROI-Informationen (32), die eine oder mehrere weitere ROI innerhalb des medizinischen Bildes (100) anzeigen, und weitere Betrachtungsdaten (23) einschließlich Betrachtungsdaten und/oder Betrachtungszeit (334) zu empfangen, mit denen die weitere ROI auf einer Anzeige angezeigt wurde, und auf deren Grundlage die weiteren Anmerkungen (31) vorgenommen wurden, und die Qualität der weiteren Anmerkungen (31) durch Vergleichen der weiteren ROI-Informationen (32) mit den ersten und/oder zweiten ROI-Informationen (12, 22) zu überprüfen und um die weiteren Betrachtungsdaten (33) zu überprüfen.

13. Computerprogramm mit Programmcode-Mitteln, um einen Computer zu veranlassen, die Schritte des Verfahrens gemäß den Ansprüchen 1 bis 9 auszuführen.

## Revendications

1. Procédé d'analyse d'image d'une image médicale (100), ledit procédé comprenant:
- recevoir une image médicale (100), des premières annotations (11) de ladite image médicale et de la première région d'intérêt, ROI, des informations (12) indiquant une première ROI à l'intérieur de ladite image médicale (100),
- recevoir des secondes annotations (21) de ladite image médicale (100), des secondes informations de ROI (22) indiquant une seconde ROI à l'intérieur de ladite image médicale (100) et des données de visualisation (23) comprenant des données d'affichage et/ou un temps de visualisation (234) avec lequel la seconde ROI a été affichée sur un afficheur et sur la base duquel les secondes annotations (21) ont été faites, et
- vérifier la qualité des secondes annotations (21) en comparant les secondes informations de ROI (22) aux premières informations de ROI (12) et en vérifiant les données de visualisation (23).

2. Procédé selon la revendication 1,
dans lequel la vérification des données de visualisation (23) consiste à vérifier si les données de visualisation (23) satisfont à une exigence de données de visualisation (40).

3. Procédé selon l'une quelconque des revendications précédentes,
dans lequel les données de visualisation (23) comprennent en outre un niveau de zoom (231) et/ou un niveau de panoramique (232) et/ou
dans lequel les données d'affichage comprennent une résolution (233) et/ou une taille d'affichage (236) et/ou un nombre de pixels (235), en particulier dans l'afficheur et/ou la seconde ROI.

4. Procédé selon les revendications 2 et 3,
dans lequel l'exigence de données de visualisation 40 comprend un seuil de temps de visualisation (44) et/ou un seuil de niveau de zoom (41) et/ou un seuil de niveau de panoramique (42) et/ou un seuil de résolution (43) et/ou un nombre de seuil de pixels (45) et/ou un seuil de taille d'affichage (46).

5. Procédé selon l'une quelconque des revendications précédentes,
dans lequel lesdites informations de ROI (12, 22) comprennent des informations de position et/ou des informations de taille indiquant la position et/ou la taille de la ROI.

6. Procédé selon l'une quelconque des revendications précédentes,
dans lequel les premières annotations (11) sont fournies par un ou plusieurs utilisateurs et/ou générées par analyse de l'image automatisée.

7. Procédé selon l'une quelconque des revendications précédentes, comprenant en outre
- délivrer le résultat de la vérification de qualité (4), et/ou
- adapter l'image (100) en fonction de la vérification de qualité (4).

8. Procédé selon l'une quelconque des revendications précédentes, comprenant en outre
- recevoir d'autres annotations (31) de ladite image médicale, d'autres informations de ROI (32) indiquant une ou plusieurs autres ROI à l'intérieur de ladite image médicale (100) et d'autres données de visualisation (33) comprenant des données d'affichage et/ou un temps de visualisation avec lequel l'autre ROI a été affichée sur un afficheur et sur la base duquel les autres annotations (31) ont été faites, et
- vérifier la qualité des autres annotations (31) en comparant les autres informations de ROI (32) aux première et/ou seconde informations de ROI (12, 22) et en vérifiant les autres données de visualisation (33).

9. Procédé selon l'une quelconque des revendications précédentes,
dans lequel les premières annotations (11) et les premières informations de ROI (12) sont basées sur la visualisation de l'image médicale (100) sur un afficheur à haute résolution et dans lequel la seconde ROI a été affichée sur un afficheur à basse résolution.

10. Système (5) d'analyse d'image d'une image médicale (100), ledit système comprenant:
- une première unité d'entrée (6) configurée pour recevoir une image médicale (100), des premières annotations (11) de ladite image médicale (100) et une première région d'intérêt, ROI, des informations (12) indiquant une première ROI à l'intérieur de ladite image médicale (100),
- une seconde unité d'entrée (7) configurée pour recevoir des secondes annotations (22) de ladite image médicale (100), des secondes informations de ROI (22) indiquant une seconde ROI à l'intérieur de ladite image médicale (100) et des données de visualisation (23) comprenant des données d'affichage et/ou un temps de visualisation (234) avec lequel la seconde ROI a été affichée sur un afficheur et sur la base duquel les secondes annotations (22) ont été faites, et
- une unité de vérification de qualité (8) configurée pour vérifier la qualité des secondes annotations (21) en comparant les secondes informations de ROI (22) aux premières informations de ROI (12) et pour vérifier les données de visualisation (23).

11. Système selon la revendication 10, comprenant en outre
- une unité de sortie (9) configurée pour délivrer le résultat de la vérification de qualité (4), et/ou
- une unité d'adaptation (10) configurée pour adapter l'image (100) en fonction de la vérification de qualité (4).

12. Système selon l'une quelconque des revendications 10 à 11,
dans lequel la seconde unité d'entrée (7) est en outre configurée pour recevoir d'autres annotations (31) de ladite image médicale (100), d'autres informations de ROI (32) indiquant une ou plusieurs autres ROI à l'intérieur de ladite image médicale (100) et d'autres données de visualisation (23) comprenant des données d'affichage et/ou un temps de visualisation (334) avec lequel l'autre ROI a été affichée sur un afficheur et sur la base duquel les autres annotations (31) ont été faites, et pour vérifier la qualité des autres annotations (31) en comparant les autres informations de ROI (32) aux première et/ou seconde informations de ROI (12, 22) et pour vérifier les autres données de visualisation (33).

13. Programme informatique comprenant des moyens de code de programme pour amener un ordinateur à exécuter les étapes du procédé selon les revendications 1 à 9.
